# EUROPEAN PATENT APPLICATION

(11) **EP 2 085 107 A1**
(43) Date of publication of application: **05.08.2009**
(21) Application number: 08396003.9
(22) Date of filing: 31.01.2008
(51) Int. Cl.: A61M 16/01, A61M 16/18, A61M 16/00, A61M 16/10

(54) **A method and system for anesthesia delivery**

(71) Applicant: GENERAL ELECTRIC COMPANY, Schenectady, NY 12345 (US)
(72) Inventor: Erkki, Heinonen, 00750 Helsinki (FI); Heikki, Haveri, 03150 Huhmari (FI)
(74) Representative: Kanerva, Arto

(57) **Abstract**

A method for delivering an anesthetic agent into a breathing air of a subjectis disclosed herein. The method for delivering an anesthetic agent into a breathing air of a subject includes feeding (21) a target anesthetic agent concentration, measuring (23) a concentration of the anesthetic agent component in the breathing air, comparing (24) the measured concentration with the fed target anesthetic agent concentration, and choosing (25) one of regulating or not regulating the delivery of the anesthetic agent into the breathing air based on the comparison to achieve the target anesthetic agent concentration. The method for delivering an anesthetic agent into a breathing air of a subject also includes delivering (26) doses of the anesthetic agent into the breathing air, adsorbing (28) the anesthetic agent and releasing (29) the absorbed anesthetic agent into the breathing air. An anesthesia delivery system is also provided.

## Description

### BACKGROUND OF THE INVENTION

This disclosure relates generally to an anesthesia delivery system and a method for delivering anesthetic agent into a breathing air of a subject.

Inhalation anesthesia agents are volatile halogenated hydrocarbon compounds. They are expensive, environmentally harmful, and long-term exposure may be dangerous. Therefore inhalation anesthesia systems are designed to minimize the use of the agents.

In inhalation anesthesia the volatile agent is vaporized from liquid form to vapor mixed in the patient's inhalation gas mixture. From the lungs the anesthesia agent dissolves into blood that transports that to brain, other organs, muscles, and fat tissue. Brain concentration is assumed to determine the depth of anesthesia. When maintaining this, all body compartments fill to the required concentration. The agents are variable in solubility to various body compartments, but relatively slow body metabolism is common characteristic of those all. Therefore, once the body compartments get filled, body agent uptake is drastically reduced to compensate only the metabolic demand.

While the body uptake is reduced, to preserve the body agent concentration, alveolar concentration has to be maintained. To facilitate this without additional agent consumption, current delivery systems use re-breathing circuit where expired breathing gas containing anesthesia agent in the alveolar concentration is re-circulated back to inspiration gas. However, before re-breathing, the expired gas needs to be cleared from patient-produced CO2 and filled with oxygen corresponding to patient 02 consumption. CO2 is removed in CO2 adsorber comprising typically NaOH or KOH soda-lime. Oxygen is added through fresh gas inlet, which is used to control the re-breathing circuit mixture. Fresh gas may contain also inhalation agent vapor mixed to it with vaporizer, or the agent is injected directly to the circuit. Another port of the circuit is connected to a ventilator controlling the circuit pressure to ventilate the patient. Through this port ventilator provides the inspiration volume that is for typical adult patient 500 ml into the breathing circuit during inspiration and receives respective volume during expiration. The ventilator comprises a volume that preserves the expiration gas for the next inspiration. Typically this ventilator preserving volume is maximum 1500 ml.

Anesthesia delivery systems where the supplied amount of fresh gas corresponds to the patient gas consumption, at steady state typically 150-200 ml/min oxygen, is called closed circuit. These have, however, many difficulties like circuit leakages including patient connection, gas monitor gas sampling stream removing gas from the circuit for analysis, accumulation of volatile organic compound (VOC) gases, and agent degradation reactions in the soda-lime adsorber. The fresh gas flow typically somewhat exceeds the patient gas uptake to allow some margin for leakages, gas sampling without need of returning the sampling gas to the circuit, and circuit ventilation out of the unwanted gas compounds. Even closed circuits are temporarily opened to wash out the contaminations. While the fresh gas flow is thus larger than the patient consumption, the circuit is provided an excess gas port to prevent unwanted pressure build-up in the circuit. This port is often actively controlled to maintain the desired circuit pressure. In typical modem low-flow inhalation anesthesia the fresh gas flow rate varies from 0.5-1.5 L/min. The higher the flow is, the more open the circuit and the more anesthesia agent is lost and vice versa.

Unnecessary to say the described inhalation anesthesia delivery system is complex and requires a lot of infrastructure. This is one aspect promoting intravenous anesthesia where the drug is infused as a bolus or continuous flow directly to a patient's vein. However, even then, possibility for inhalation anesthesia delivery is often preserved as a backup for the intravenous injection and for patients developing some allergic reaction to the intravenous drugs. Therefore, inhalation system complexity is present even in intravenous anesthesia. Inhalation anesthesia is increasingly used also in intensive care departments to sedate the patients. However, the ICU departments lack totally the required system infrastructure and often also room for that. Other places where inhalation anesthesia demand exists but infrastructure is often missing are all kind of field conditions and developing countries. Also commercially available for limited inhalation agents are inhalation anesthesia delivery systems having the anesthesia agent preserving capability comparable with state of the art anesthesia practice. This can be provided using agent reflector connected to airway tube in the reciprocating gas flow path between the patient and circuit Y-piece. There the reflector adsorbs agent from exhalation gas and releases to inspiration gas. Such provide agent preserve capacity respective to 1-1.5 L/min fresh gas flow of the re-breathing system. Using the reflector removes the need for CO2 adsorber when the expired gas is not circulated back to inspiration. Furthermore, also fresh gas mixer is eliminated, as ventilator when controlling the circuit pressure provides the breathing gas.

When the reflector prevents the anesthesia agent flow-through, the agent needs to be delivered into- or between the reflector and the patient. The agent may be administered e.g. as liquid infusion, as gasified on anesthetic vaporizer, as aerosol droplets produced with a nebulizer, or as a bolus source embedded in the system that can be activated to release the agent. Liquid infusion and nebulizer has been used to deliver the anesthetic agent. A side-stream infrared gas analyzer suctions gas flow from breathing circuit through sampling line into the IR sensor for concentration measurement. The gas sampling system comprising a pump, flow regulation systems, and sampling gas flow path, however, increases system size and complexity for the needs of compact anesthesia delivery system and therefore is not practical in operating room environment. Further gas analyzer is fitted to measure inspired gas concentration and using this to control the gas delivery in order to achieve a desired gas mixture at the patient. Using inspired gas concentration to control the mixture at the patient is, however, even at its best very slow and target would be hardly ever achieved. So it fails in controlling the state of the patient.

Independently of the anesthetic agent delivery method, accurate delivery of the very small amounts of agent is difficult. Using the reflector reduces the delivered amount further by reducing the gas volume the agent is delivered into.

### BRIEF DESCRIPTION OF THE INVENTION

The above-mentioned shortcomings, disadvantages and problems are addressed herein which will be understood by reading and understanding the following specification.

In an embodiment, an apparatus for an anesthesia delivery system includes an airway tube" an anesthetic agent dosing unit, an anesthetic agent adsorber unit and a gas analyzing apparatus. The anesthesia delivery system also includes a user interface and an anesthetic agent delivery controller connected to the gas analyzing apparatus for receiving measurement results and also connected to the user interface for receiving target anesthetic agent concentration and further connected to the anesthetic agent dosing unit for controlling an anesthetic agent delivery based on received measurement results and the target anesthetic agent concentration. Each of the anesthetic agent dosing unit, the anesthetic agent adsorber unit and the gas analyzing apparatus is adapted to be mounted on the airway tube.

In another embodiment, a method for delivering an anesthetic agent into a breathing air of a subject includes feeding a target anesthetic agent concentration, measuring a concentration of the anesthetic agent component in the breathing air and comparing the measured concentration with the fed target anesthetic agent concentration. The method for delivering an anesthetic agent into a breathing air of a subject also includes choosing one of regulating or not regulating the delivery of the anesthetic agent into the breathing air based on the comparison to achieve the target anesthetic agent concentration, delivering doses of the anesthetic agent into the breathing air, adsorbing the anesthetic agent and releasing the absorbed anesthetic agent into the breathing air.

In yet another embodiment, a method for delivering an anesthetic agent into a breathing air of a subject includes feeding a target anesthetic agent concentration, measuring a concentration of the anesthetic agent component in the breathing air, comparing the measured concentration with the fed target anesthetic agent concentration and choosing one of regulating or not regulating the delivery of anesthetic agent into the breathing air based on the comparison to achieve the target anesthetic agent concentration. A method for delivering an anesthetic agent into a breathing air of a subject also includes delivering doses of the liquid anesthetic agent into the breathing air, vaporizing the anesthetic agent, adsorbing the anesthetic agent from the breathing air into an anesthetic agent adsorber unit during an exhalation phase and releasing the anesthetic agent into the breathing air during an inhalation phase.

Various other features, objects, and advantages of the invention will be made apparent to those skilled in the art from the accompanying drawing and detailed description thereof.

### BRIEF DESCRITION OF THE DRAWINGS

Figure 1 is a general view of a system in accordance with an embodiment; and

Figure 2 is a block diagram illustrating a method in accordance with an embodiment.

### DETAILED DESCRIPTION OF THE INVENTION

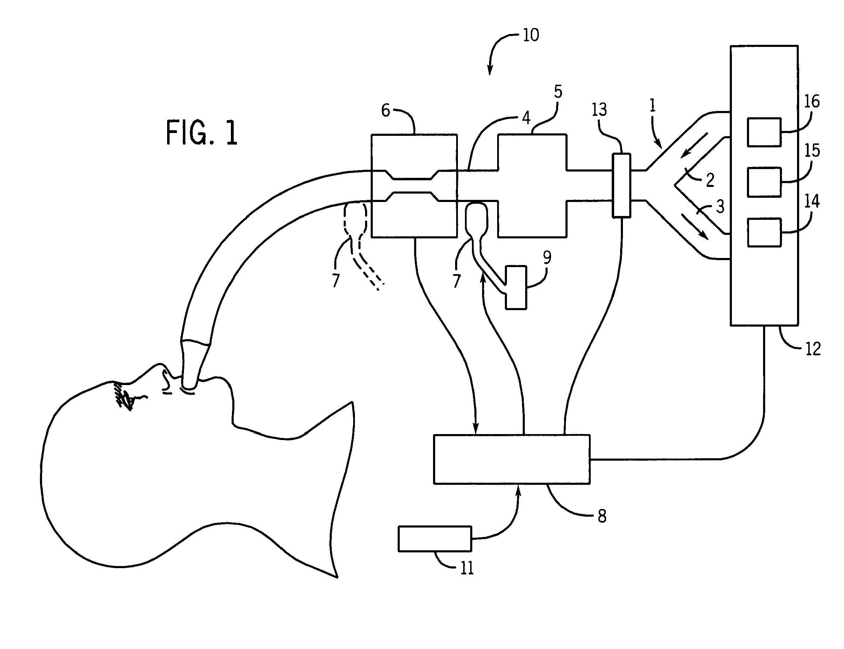

Figure 1 shows an anesthesia delivery system 10 comprising a breathing connector 1, such as Y-piece, having three branches. The first branch is an inhalation tube 2 for delivery of inhaled gases during inspiration, the second branch is an exhalation tube 3 carrying during expiration expired gas and the third branch is an airway tube 4 carrying during inspiration the inhaled gas to and during the expiration the expired gas from the subject. Also the anesthesia delivery system 10 comprises an anesthetic agent adsorber unit 5, such as a reflector, for adsorbing anesthetic agent usually when a subject is exhaling and for releasing this agent into inspiratory breathing air. Further the anesthesia delivery system 10 comprises a gas analyzing apparatus 6 and an anesthetic agent dosing unit 7, which both are assembled into the airway tube 4. The gas analyzing apparatus 6, such as an infrared gas analyzer, is for measuring a concentration of at least one component of a gas flowing along the airway tube 4. The anesthetic agent dosing unit 7, such as a nebulizer or syringe injector, is for dosing anesthetic agent into the breathing air flowing along the airway tube 4. The anesthesia delivery system also comprises an anesthetic agent delivery controller 8 and a user interface 11. The anesthetic agent delivery controller 8 is adapted to control a liquid delivery into the breathing air and to control a liquid delivery from a liquid reservoir 9 to the anesthetic agent dosing unit 7. Further the anesthetic agent delivery controller 8 is adapted to receive measurement results from the gas analyzing apparatus and also to receive a target anesthetic agent concentration from a user interface 11.

In Figure 1 a breathing circuit represents an open one. For a control of a subject's breathing there is a ventilator 12 connected to the inhalation tube 2 and the exhalation tube 3 of the breathing connector 1. The ventilator 12 comprises an inspiration controller 14 that regulates the breathing gas mixture and flow rate for the breathing gas delivery to the subject, and an expiration controller 15 regulating the subject's expiration flow and pressure. The ventilator further comprises a timing unit 16 to control a duration of the inspiration and expiration.

The liquid delivery by means of the anesthetic dosing unit 7 may happen by nebulizing small or preferably micro droplets into the brerathing air. Because of their small size, large surface area, and high vapor pressure of the volatile anesthesia agents, these are vaporized into the breathing gas rapidly and spontaneously before inhaled into the subject's breathing. If the anesthesia agent is delivered into contact with the breathing gas as liquid with injection technique using a syringe and syringe pump actuator, the vaporization is not as effective as with micro droplets because of larger liquid volume ratio to surface area. In these circumstances special actions may be needed for vaporization. These may comprise a heated surface (not shown) where the liquid is deliverd on and vaporized with the aid of the heat. Alternatively the anesthetic agent can be injected into the anesthetic agent adsorber unit 5 for vaporization into the breathing gas passing the adsorber unit 5 during inspiration.

The target anesthetic agent concentration fed to the user interface 11 may be a single value or a range having minimum and maximum values. The anesthetic agent delivery controller 8 is adapted to adjust anesthetic agent delivery into breathing air flowing along the airway tube 4 based on the measurement results from the gas analyzing apparatus 6 and the target anesthetic agent concentration in order to match the measurement result with the target anesthetic agent concentration. The anesthetic agent delivery controller 8 receives power e.g. from a separate power source not shown in the figure, or from a ventilator 12.

Preserving the anesthetic agent in the subject's breathing gas effectively, the decrease of the gas concentration after stopping the delivery is slow. To make the control easier, the user given target concentration may also have a time forecast when the anesthesia is likely to stop. Using this information the system can begin preparation in reducing the delivery to obtain the given target at given time.

The anesthetic agent dosing unit 7 is advantageously of a mesh plate type comprising an array of miniature micrometer-range holes vibrated with an ultrasound crystal coupled to the mesh plate. While vibrating, the mesh plate may be arranged to act as a pump as well as providing the liquid feed action to the supply. Alternatively, the liquid feed may be controlled through pumping action control or control of a valve connected in the liquid feed line connected to pressurized liquid reservoir 9. It is advantageous that the generated aerosol comprises droplets small enough to allow passive vaporization when in contact to the breathing gas. For this purpose the droplets shall be less than 10 micrometers, preferably less than or equal to 5 micrometers. The droplet size produced corresponds to the hole size of the mesh plate. It is also advantageous that the liquid delivered into contact with the breathing gas is vaporized as rapidly as possible. Delays in vaporization will delay in the breathing gas concentration change, and respectively delaying the measured response. Such delays make the concentration control more difficult.

The anesthetic agent dosing unit 7 may be connected upstream (solid line) or downstream (dashed illustration) of the gas analyzing apparatus 6. The delivery may also be synchronized with patient breathing to deliver either during inspiration or expiration. For this purpose the anesthesia delivery system may be equipped also with a flow phase detector 13 to detect the subject flow phase, such as a flow sensor, or this same information can be acquired from the ventilator 12 including the flow phase detector 13 to detect the subject flow phase. Further, if the gas analyzing apparatus 6 is fitted to measure also patient breathing gas CO2 concentration, that can be applied on the purpose as well. In this case the expiration phase is identified when the measured CO2 concentration exceeds given CO2 value and respectively during inspiration is below given CO2 value. Delivery synchronization corresponds with the delivery point location advantageously so that the aerosol delivered does not end up to the measurement point immediately upon delivery to avoid disturbing uninformative measurement noise caused by high-concentration delivery doses. Upstream connection would then correspond to synchronization with expiration phase and would provide the longest time for the vaporization. Downstream delivery would then be synchronized with the inspiration phase respectively. In the former case the delivery dose would first be adsorbed in the anesthetic agent adsorber unit 5 and then released to the coming inspiration flow. In the latter case the dose becomes diluted in the alveolar volume of the subject.

A result of the system described is very small and compact system when each of the anesthetic agent dosing unit 7, the anesthetic agent adsorber unit 5 and said gas analyzing apparatus 6 is mounted on the airway tube 4. All extra wires or tubes connecting the system to any outside device are avoided making a working environment around patient more safe from both patient's and personnel's point of view.

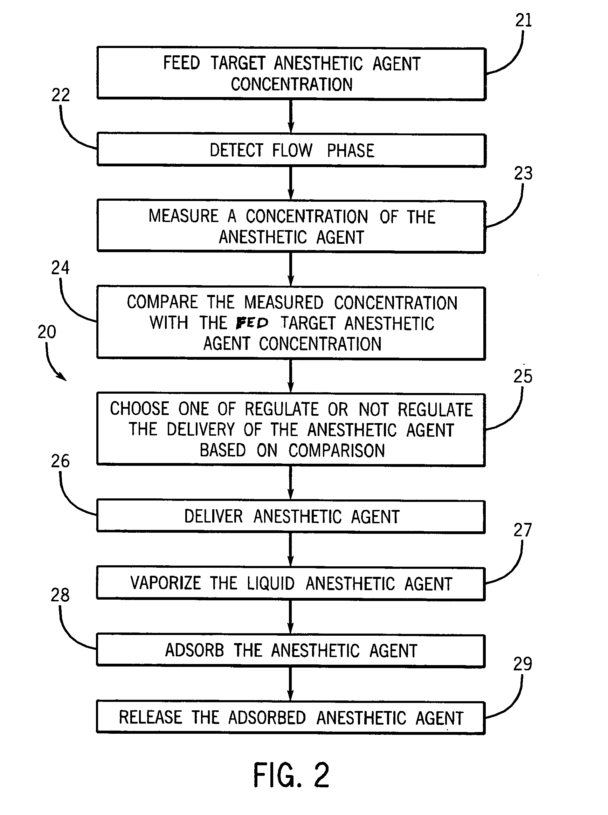

Figure 2 shows a method 20 for delivering anesthetic agent into a breathing air of a subject. References to figure 1 has also been made while discussing the method steps.

A target anesthetic agent concentration is fed to the user interface 11 at step 21 to set a concentration that should be achieved and maintained during anesthesia.

Step 22 is an optional step to detect the subject's flow phase which may be either an inspiration or an expiration and which flow phase information can used while anestetizing the subject. The flow phase can be detected by means of the flow phase detector 13.

At step 23 when the patient is exhaling the concentration of the anesthetic agent in the breathing air exhaled is measured by means of the gas analyzing apparatus 6, which measuring is made in the airway tube 4. Thus this measuring is made immediately without withdrawing the sample of gas from breathing air. Measuring in the airway tube avoids any extra connections to outside monitors and makes the system compact and more reliable. The benefit for measuring exhaled gas compared to inspired gas is the fact that this gives a reliable information about the state of the patient.

At step 24 the measured anesthetic agent concentration of step 23 is compared by the anesthetic agent delivery controller 8 with the fed target anesthetic agent concentration of step 21 to find out whether or not the anesthetic agent concentration of the breathing air exhaled by the patient corresponds to the target anesthetic agent concentration.

At step 25 the anesthetic agent delivery controller 8 chooses either to regulate or not regulate the delivery of the anesthetic agent into the breathing air of the patient. The decision to choose to regulate or to not regulate depends on the comparison made between the measured anesthetic agent concentration of the breathing air exhaled and the target anesthetic agent concentration. If the comparison shows that the measured anesthetic agent concentration corresponds to the target anesthetic agent concentration or is above, then no anesthetic agent will be delivered into the breathing air. Instead, if the comparison shows that the measured anesthetic agent concentration is lower than the target anesthetic agent concentration, then the anesthetic agent will be delivered. The decision to regulate may mean to deliver a fixed amount of anesthetic agent per breath whereas not regulate may mean that no anesthetic agent is delivered to a breath. Alternatively, these regulate and not regulate may mean increasing and decreasing the amount of anesthetic agent delivered per breath respectively.

Doses of an anesthetic agent are delivered at step 26 into the breathing air by means of the anesthetic agent dosing unit 7 depending on the decision chosen at step 25. The delivery may happen either during expiration phase or inspiration phase and which information can be obtained by detecting the subject's flow phase as explained at step 22. Injecting liquid into breathing air should be advantageously made during expiration phase, but aerosol delivery can happen either during inspiration or expiration phase. Therefore the information from flow phase detector 13 can be used to detect whether the patient is inhaling or exhaling.

Step 27 is an optional step wherein the anesthetic agent is delivered into the breathing air in bigger liquid doses and thus needs to be effectively vaporized. While nebulizing the liquid anesthetic agent is already as an aerosol in the breathing agent, but when injecting liquid volumes more time of efforts are needed to vaporize.

The anesthetic agent which may be in a liquid form or vaporized is adsorbed at step 28 into the anesthetic agent adsorber unit 5. The anesthetic agent may flow with the breathing air while the patient is exhaling or it may be delivered directly into the anesthetic agent adsorber unit 5 by means of the anesthetic agent dosing unit 7.

When the patient is inhaling at step 29 the adsorbed anesthetic agent of the anesthetic agent adsorber unit 5 is released into the breathing air flowing towards the patient for the breathing.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. An anesthesia delivery system comprising:
an airway tube (4);
an anesthetic agent dosing unit (7);
an anesthetic agent adsorber unit (5);
a gas analyzing apparatus (6);
a user interface (11); and
an anesthetic agent delivery controller (8) connected to said gas analyzing apparatus (6) for receiving measurement results and also connected to said user interface (11) for receiving the target anesthetic agent concentration and further connected to said anesthetic agent dosing unit (7) for controlling an anesthetic agent delivery based on received measurement results and the target anesthetic agent concentration,
wherein each of said anesthetic agent dosing unit (7), said anesthetic agent adsorber unit (5) and said gas analyzing apparatus (6) is adapted to be mounted on said airway tube (4).

2. An anesthesia delivery system according to claim 1, wherein said gas analyzing apparatus (6) is an infrared gas analyzer.

3. An anesthesia delivery system according to claim 1, wherein said anesthetic agent adsorber unit (5) is adapted to adsorb the anesthetic agent in the breathing air exhaled and to release the adsorbed anesthetic agent to the breathing air inhaled.

4. An anesthesia delivery system according to claim 1 or 3, wherein said anesthetic agent adsorber unit (5) is adapted to adsorb the anesthetic agent in the breathing air exhaled and the anesthetic agent delivered by said anesthetic agent dosing unit (7) and to release the adsorbed anesthetic agent to the breathing air inhaled.

5. An anesthesia delivery system according to claim 1, wherein said anesthetic agent dosing unit (7) is adapted to be mounted on said airway tube (4) between said gas analyzing apparatus (6) and said anesthetic agent adsorber unit (5).

6. An anesthesia delivery system according to claim 1, wherein said gas analyzing apparatus (6) is adapted be mounted on said airway tube (4) between said anesthetic agent dosing unit (7) and said anesthetic agent adsorber unit (5).

7. An anesthesia delivery system according to claim 1, wherein said gas analyzing apparatus (6), said anesthetic agent dosing unit and said anesthetic agent adsorber unit (5) are adapted to be mounted on said airway tube one after another.

8. An anesthesia delivery system according to claim 1, wherein said anesthetic agent dosing unit (7) is adapted to deliver small liquid droplets into breathing air.

9. An anesthesia delivery system according to claim 1, wherein said anesthetic agent dosing unit (7) is adapted to deliver an injected liquid anesthetic agent into the breathing air.

10. An anesthesia delivery system according to claim 1, wherein said gas analyzing apparatus (6) is adapted to measure the gas concentration of an anesthetic agent component in the breathing air expired and which measurement value is fed to said anesthetic agent delivery controller (8) for controlling the anesthetic agent delivery to adjust the measured gas concentration of the anesthetic agent to correspond with the target anesthetic agent concentration.

11. An anesthesia delivery system according to claim 1 or 10, wherein the target anesthetic agent concentration may be a single value or a concentration range having minimum and maximum values.

12. An anesthesia delivery system according to claim 1, further comprising a flow phase detector (13) to detect a subject flow phase.

13. An anesthesia delivery system according to claim 12, wherein said flow phase detector (13) to detect the subject flow phase is a flow sensor.

14. An anesthesia delivery system according to claim 12, wherein said flow phase detector (13) to detect the subject flow phase is included in a ventilator (12).

15. An anesthesia delivery system according to claim 1, wherein said gas analyzing apparatus (6) is adapted to measure breathing gas carbon dioxide concentration to detect a subject's flow phase.

16. A method for delivering anesthetic agent into a breathing air of a subject comprising:
feeding (21)a target anesthetic agent concentration;
measuring (23) a concentration of an anesthetic agent component in the breathing air;
comparing (24) the measured concentration with the fed target anesthetic agent concentration;
choosing (25) one of regulating or not regulating the delivery of the anesthetic agent into the breathing air based on said comparison to achieve the target anesthetic agent concentration;
delivering (26) doses of an anesthetic agent into the breathing air;
adsorbing (28) the anesthetic agent; and
releasing (29) the absorbed anesthetic agent into the breathing air.

17. A method according to claim 16, wherein said releasing (29) is made during an inhalation phase.

18. A method according to claim 16, wherein said delivering (26) doses of anesthetic agent into a breathing air includes delivering a liquid anesthetic agent.

19. A method according to claim 18, further comprising vaporizing (27) the liquid anesthetic agent in the breathing air.

20. A method according to claim 16, wherein said adsorbing (28) anesthetic agent includes adsorbing the anesthetic agent from the breathing air into an anesthetic agent adsorber unit (5) during an exhalation phase.

21. A method according to claim 16, wherein said measuring (23) includes measuring the anesthetic agent concentration of the flowing breathing air expired and which anesthetic agent concentration is used for adjusting anesthetic agent delivery.

22. A method according to claim 16, further comprising detecting (22) a subject's flow phase which may be one of an inspiration or an expiration and which flow phase information is used for said delivering doses of a liquid anesthetic agent into the breathing air.

23. An anesthesia delivery system comprising:
an airway tube (4) for flowing a breathing air including breathing air inhaled and exhaled;
an anesthetic agent dosing unit (7) for dosing an anesthetic agent into a subject's breathing air;
an anesthetic agent adsorber unit (5) for adsorbing the anesthetic agent and for releasing the anesthetic agent into the breathing air inhaled;
a gas analyzing apparatus (6) for measuring an anesthetic agent component concentration in the breathing air;
a user interface (11) for feeding a target anesthetic agent concentration; and
an anesthetic agent delivery controller (8) connected to said gas analyzing apparatus (6) for receiving measurement results and
also connected to said user interface (11) for receiving the target anesthetic agent concentration and further connected to said anesthetic agent dosing unit (7) for controlling an anesthetic agent delivery based on received measurement results and the target anesthetic agent concentration,
wherein each of said anesthetic agent dosing unit (7), said anesthetic agent adsorber unit (5) and said gas analyzing apparatus (6) is adapted to be mounted on said airway tube (4).
